# EUROPEAN PATENT SPECIFICATION

(11) **EP 2 170 854 B1**
(45) Date of publication and mention of the grant of the patent: **24.10.2012**
(21) Application number: 08777692.8
(22) Date of filing: 23.06.2008
(51) Int. Cl.: C07D 301/04, C07D 301/06, C07D 303/04

(54) **METHOD FOR PRODUCING PROPYLENE OXIDE**
VERFAHREN ZUR HERSTELLUNG VON PROPYLENOXID
PROCÉDÉ POUR PRODUIRE DE L'OXYDE DE PROPYLÈNE

(30) Priority: 27.06.2007 JP 2007168693; 28.03.2008 JP 2008086236
(43) Date of publication of application: 07.04.2010
(73) Proprietor: Sumitomo Chemical Company, Limited, Tokyo 104-8260 (JP)
(72) Inventor: KANAZAWA, Hideo, Toyonaka-shi Osaka 561-0802 (JP); MIZUNO, Masahiko, Nara-shi Nara 630-8114 (JP); YAMAMOTO, Michio, Otsu-shi Shiga 520-0246 (JP)
(74) Representative: Vossius & Partner
(86) International application number: PCT/JP2008/061786
(87) International publication number: WO 2009/001948

(56) References cited:
- GB-A- 2 008 113
- US-A- 4 390 738
- US-A- 5 849 937
- GREGOR JENZER, TAMAS MALLAT, MAREK MACIEJEWSKI, FLORIAN EIGENMANN, ALFONS BAIKER: "Continuous epoxidation of propylene with oxygen and hydrogen on a Pd?Pt/TS-1 catalyst" APPLIED CATALYSIS A: GENERAL, no. 208, 2001, pages 125-133, XP002497491
- LAUFER W ET AL: "Direct oxidation of propylene and other olefins on precious metal containing Ti-catalysts" APPLIED CATALYSIS A: GENERAL, ELSEVIER SCIENCE, AMSTERDAM, NL, vol. 213, 1 January 2001 (2001-01-01), pages 163-171, XP002268789 ISSN: 0926-860X
- MEIERS R ET AL: "Synthesis of Propylene Oxide from Propylene, Oxygen, and Hydrogen Catalyzed by Palladium-Platinum-Containing Titanium Silicalite" JOURNAL OF CATALYSIS, ACADEMIC PRESS, DULUTH, MN, US, vol. 176, no. 2, 10 June 1998 (1998-06-10), pages 376-386, XP004447349 ISSN: 0021-9517
- R. MEIERS AND W.F. HÖLDERICH: "Epoxidation of propylene and direct synthesis of hydrogen peroxide by hydrogen and oxygen" CATALYSIS LETTERS, no. 59, 1999, pages 161-163, XP002497495

## Description

The present invention relates to a method for producing propylene oxide by reaction of propylene, hydrogen, and oxygen, in an acetonitrile solvent or in a mixture of solvents which include acetonitrile and water, in presence of a titanosilicate catalyst having a 12 or more-membered oxygen ring pore and a palladium catalyst supported on a carrier, wherein the propylene is fed into the reaction in the form of liquefied propylene.

FY2002 Report of Development of Non-halogen Chemical Process Technology and Development of Next-Generation Chemical Process Technology (pages 161 and 175, 2003) discloses a method for producing propylene oxide by reaction of hydrogen, oxygen, and propylene gas, in a mixture of solvents which include acetonitrile and water, in the presence of a noble metal catalyst and a titanosilicate catalyst.

Jenzer et al. describe the continuous epoxidation of propylene with oxygen and hydrogen on a Pd-Pt/TS-1 catalyst (Jenzer G., Applied Catalysis A, 2001, 208:125-133).

Laufer et al. report on the direct epoxidation of propylene and other olefins on precious metal containing Ti-catalysts (Laufer W., Applied Catalysis A, 2001, 213:163-171).

Meiers et al. describe the synthesis of propylene oxide from propylene, oxygen, and hydrogen catalyzed by palladium-platinum-containing titanium silicalite (Meiers R., Journal of Catalysis, 1998, 198, 176:376-386).

Meiers et al. describe the epoxidation of propylene and direct synthesis of hydrogen peroxide by hydrogen and oxygen (Meiers R., Catalysis Letters, 1999, 59:161-163).

US 4 390 738 A discloses the production of olefine oxides or derivatives thereof by reacting an olefinic compound, e.g., ethylene, propylene or butadiene with oxygen in the presence of a catalyst containing copper bonded to a peroxo group.

US 5 849 937 A describes a method of operating an olefin epoxidation facility comprised of a serially connected cascade of at least two fixed bed reactors each containing a heterogeneous catalyst.

GB 2 008 113 A discloses the co-oxidation of an olefin and a beta-substituted saturated aliphatic aldehyde.

The method described in FY2002 Report of Development of Non-halogen Chemical Process Technology and Development of Next-Generation Chemical Process Technology (pages 161 and 175, 2003), however, has not been always industrially efficient in terms of productivity.

In view of the aforementioned problem, an object of the present invention is to provide a method for producing propylene oxide, including: reacting hydrogen, oxygen, and propylene, in an acetonitrile solvent or in a mixture of solvents which include acetonitrile and water, in the presence of a titanosilicate catalyst having a 12 or more-membered oxygen ring pore and a palladium catalyst supported on a carrier, wherein the propylene is fed into the reaction in the form of liquefied propylene.

For a fuller understanding of the nature and advantages of the invention, reference should be made to the ensuing detailed description.

Propylene for use in reaction of the present invention is produced, for example, by thermolysis, heavy oil catalytic cracking, or methanol catalytic reforming. The propylene is either purified propylene or crude propylene not having undergone a purification process. The propylene is generally 90% or greater by volume, and preferably 95% or greater by volume. The propylene may also include, for example, propane, cyclopropane, methyl acethylene, propadiene, butadiene, butanes, butenes, ethylene, ethane, methane, or hydrogen, other than propylene.

The reaction of the present invention involves liquefied propylene. The propylene is preferably premixed with and thereby dissolved in an acetonitrile solvent or in a mixture of solvents which include acetonitrile and water before being fed into a reactor; alternatively, the liquefied propylene by itself may be fed into the reactor. The propylene for use in the reaction may also include gas such as nitrogen gas and hydrogen gas.

The reaction of the present invention involves the acetonitrile solvent or the mixture of solvents which include acetonitrile and water. The weight ratio of water to acetonitrile is in a range from (i) 0 : 100 to (ii) 50 : 50, preferably in a range from (iii) 21 : 79 to (iv) 40 : 60.

The amont of the solvent to be fed is generally from 0.02 to 70 parts by weight, preferably from 0.2 to 20 parts by weight; more preferably, from 1 to 10 parts by weight per part by weight of propylene feed.

The acetonitrile may be crude acetonitrile produced as a byproduct in acrylonitrile production process, or alternatively purified acetonitrile. Generally, purified acetonitrile is used. The purity is usually 95% or greater, and preferably 99% or greater; more preferably, 99.9% or greater. The crude acetonitrile typically includes water, acetone, acrylonitrile, oxazole, allyl alcohol, propionitrile, hydrocyanic acid, ammonia, and/or a trace amount of copper or iron, other than acetonitrile.

The oxygen in molecular form may be oxygen purified by a cryogenic separation, oxygen purified by a pressure swing adsorption (PSA), or air. The amount of oxygen to be fed is generally from 0.005 to 10 mol, preferably from 0.05 to 5 mol per mol of the propylene to be fed. A production method of the hydrogen is not particularly limited; for example, steam reforming of a hydrocarbon. Generally, hydrogen having 80% or greater by volume, and preferably 90% by volume is used. The amount of hydrogen to be fed is from 0.05 to 10 mol, preferably from 0.05 to 5 mol per mol of propylene.

Generally, a gas composition of the hydrogen and the propylene is preferably out of an explosibility range for the sake of safety; thus, inclusion of a dilution gas is preferable for the reaction. Examples of the dilution gas encompass nitrogen gas, argon gas, methane gas, ethane gas, propane gas, and carbon dioxide gas. Among the above, the nitrogen gas and the propane gas are preferable; the more preferable thereof is the nitrogen gas. In a case where a concentration of the hydrogen is controlled so that the gas composition is out of the explosibility range, the hydrogen concentration in a feed gas is generally required to be 3.9% or less by volume. In this case, the concentration of oxygen is only required to be not greater than a limiting oxygen concentration of the propylene, i.e. generally 11.5% or less by volume, and preferably 9% or less by volume. The dilution gas is fed so that such a composition is achieved. In a case where the oxygen concentration is controlled so that the gas composition is out of the explosibility range, the oxygen concentration in the feed gas is generally required to be 4.9% or less by volume, and preferably 4% or less by volume. In this case, neither the hydrogen concentration nor a propylene concentration is particularly limited; generally, both the hydrogen and propylene concentrations are 10% or less by volume. The dilution gas is fed so that such a composition is achieved.

It is preferable that a volume ratio of the oxygen to the hydrogen contained in the gas at an outlet of the reactor be not greater than 3.5. This suppresses an amount of a propane byproduct. A lower limit of the volume ratio is not particularly set; generally, it is 0.01, and preferably 0.1. The volume ratio of the oxygen to the hydrogen contained in the gas at the outlet of the reactor is set by controlling a volume ratio of the oxygen to the hydrogen contained in the gas at an inlet of the reactor, in accordance with a result of measurement of (i) the volume ratio of the oxygen to the hydrogen contained in the gas at the inlet of the reactor and (ii) that of the oxygen to the hydrogen contained in the gas at the outlet of the reactor.

The titanosilicate catalyst having a 12 or more-membered oxygen ring pore can be any porous silicate with part of Si thereof substituted by Ti; for example, crystalline titanosilicate, lamellar titanosilicate, mesoporous titanosilicate, or the like. Examples of the crystalline titanosilicate encompass (i) TS-2 having MEL structure (according to the structure code of the International Zeolite Association (IZA); hereinafter the same applies), (ii) Ti-ZSM-12 having MTW structure (see Zeolites 15, 236-242 (1995)), (iii) Ti-Beta having BEA structure (see Journal of Catalysis 199, 41-47 (2001)), (iv) Ti-MWW having MWW structure (see Chemistry Letters, 774-775 (2000)), (v) Ti-UTD-1 having DON structure (see Zeolites 15, 519-525 (1995)), and (vi) TS-1 having MFI structure (see Journal of Catalysis, 130, (1991), 1-8). An example of a lamellar titanosilicate encompassed is Ti-YNU (see Angewante Chemie International Edition 43, 236-240 (2004)) Ti-MWW precursors are disclosed in Japanese Unexamined Patent Application Publication No.327425/2003 (Tokukai 2003-32745). Examples of the mesoporous titanosilicate encompass (i) Ti-MCM-41 (see Microporous Material 10, 259-271 (1997)), (ii) Ti-MCM-48 (see Chemical Communications 145-146 (1996)), (iii) Ti-SBA-15 (see Chemistry of Materials 14, 1657-1664 (2002)), and (iv) Ti-MMM-1 (see Microporous and Mesoporous Materials 52, 11-18 (2002)). The crystalline titanosilicate and the lamellar titanosilicate are preferred. Examples of the crystalline titanosilicate having a 12 or more-membered oxygen ring pore encompass the Ti-ZSM-12, the Ti-MWW, and the Ti-UTD-1. Examples of the lamellar titanosilicate having a 12 or more-membered oxygen ring pore encompass the Ti-YNU; the more preferable thereof is Ti-MWW .

The titanosilicate catalyst may be such that a silanol group thereof is silylated by a silylating agent. Examples of the silylating agent encompass 1,1,1,3,3,3-hexamethyldisilazane, trimethylsilyl chloride, and triethylsilyl chloride. The titanosilicate catalyst is generally pretreated with a hydrogen peroxide solution before use. A concentration of the hydrogen peroxide solution is in a range from 0.0001% by weight to 50% by weight. The titanosilicate catalyst preferable for the silylation includes the Ti-MWW and the Ti-MWW precursor. The silylation reduces a level of conversion of the propylene oxide into propylene glycol.

Examples of the carrier on which the palladium may be supported generally encompass (i) an oxide such as silica, alumina, titania, zirconia, and niobia, (ii) a hydrate such as niobic acid, zirconium acid, tungsten acid, and titanium acid, (iii) carbon as in activated carbon, carbon black, graphite, and carbon nanotube, and (iv) titanosilicate. Preferable carriers are the activated carbon, the Ti-MWW and the Ti-MWW precursor. From a viewpoint of chemical engineering, one kind of catalyst having multiple functions, rather than multiple kinds of catalysts having different specific gravities, is preferably provided in the reactor so that better uniformity of catalyst dispersion in the reactor is achieved. In consideration of such a viewpoint, the Ti-MWW and the Ti-MWW precursor are preferable carriers. Mixing of (i) a palladium complex or an aqueous palladium colloid solution with (ii) the Ti-MWW or the Ti-MWW precursor causes the palladium to be supported. Subsequently, a water in the mixture is removed, generally by filtration or evaporation, whereby palladium-carrying Ti-MWW or a palladium-carrying Ti-MWW precursor is produced. Examples of the palladium complex encompass tetraamminepalladium chloride. The solution including palladium colloid may be any solution having palladium particles dispersed; generally, the aqueous palladium colloid solution is used. A concentration of the palladium colloid is not particularly limited. A process of supporting the palladium on the carrier is generally performed at temperatures from 0 to 100°C, and preferably at temperatures from 20 to 60°C. Generally, use of the palladium complex is preferably combined with reduction treatment. In consideration of selectivity and reaction rate, the palladium is preferably supported on the Ti-MWW precursor. This increases production rate of the propylene oxide and reduces the level of conversion of the propylene oxide into the propylene glycol.

In a case where one of the carriers other than the titanosilicate is used, the palladium can be impregnated on the carrier after preparation of a palladium colloid solution; alternatively, the palladium salt is impregnated on the carrier after the palladium salt is dissolved in a solvent. Examples of the palladium salt encompass palladium chloride, palladium nitrate, palladium sulfate, palladium acetate, and tetraamminepalladium chloride. In a case where a colloid solution is used for supporting the palladium, generally, the catalyst is preferably calcinated under an inert gas atmosphere. In a case where the palladium salt is used for supporting the palladium, generally, the catalyst is reacted with a reducing agent either in a liquid phase or in a gas phase before use. In a case where the tetraamminepalladium chloride is used, the catalyst may be reduced in the presence of an inert gas by ammonia that is produced by thermolysis of tetraammine palladium chloride.

In a case where the palladium is supported on one of the carriers other than the titanosilicate, an amount of the palladium to be supported is generally in a range from 0.01 % to 20% by weight with respect to the catalyst, and preferably in a range from 0.1% to 5% by weight. In a case where the palladium is supported on the titanosilicate, the above amount is generally in a range from 0.001% to 5% by weight, and preferably in a range from 0.01% to 0.5% by weight.

The palladium catalyst supported on the carrier may include one or more kinds of noble metals other than the palladium. Examples of the noble metal other than the palladium encompass platinum, ruthenium, rhodium, iridium, osmium, and gold. An amount of the noble metal other than the palladium to be included is not particularly limited.

It is preferable to keep the amount of the palladium in the reactor at a certain level or higher so that catalytic performance of the catalyst is maintained. For example, in a case where the palladium is supported on one of the carriers other than the titanosilicate, a weight ratio of the palladium contained in the reactor to a reaction solvent is preferably greater than 13 ppm by weight. In a case where the palladium is carried by the titanosilicate, the weight ratio of the palladium contained in the reactor to the reaction solvent is preferably greater than 4 ppm by weight. The palladium is used in the above preferable ranges because the catalytic performance may decrease if the ratio is lower than the lower thresholds. An upper threshold thereof is not particularly set; however, an excessive amount of the palladium may cause, before the desired reaction, decomposition of generated hydrogen peroxide. Thus, the amount of the palladium is generally 3000 ppm by weight, and preferably 1000 ppm by weight.

The reaction can be performed by such methods as a batch process, a slurry-bed continuous flow process, or a fixed-bed continuous flow process; among the above, the slurry-bed continuous flow process and the fixed-bed continuous flow process are preferable in terms of productivity. According to the slurry-bed continuous flow process, the titanosilicate catalyst and the palladium catalyst supported on the carrier are filtered by a filter that is provided inside or outside the reactor, and then remain in the reactor. Subsequently, part of the catalysts remained in the reactor is continuously or intermittently taken out and then regenerated. Thereafter, the reaction may be performed while the regenerated catalysts are resupplied to the reactor; alternatively, the reaction may be performed while part of the catalysts is taken out of the system, and the titanosilicate catalyst and the palladium catalyst supported on the carrier are newly supplied to the reactor in an amount equivalent to an amount of the part of the catalysts taken out. An amount of the catalysts contained in the reactor is generally in a range from 0.01% to 20% by weight, and preferably in a range from 0.1% to 10% by weight.

According to the fixed-bed continuous flow process, the reaction is performed while reaction and regeneration treatments are repeated alternately. In this case, the catalysts are preferably molded by a molding agent or the like.

A reaction temperature is generally set in a range from 0 to 150°C, and preferably in a range from 20 to 100°C; more preferably, in a range from 40 to 70°C.

A reaction pressure is generally in a range from 0.6 to 20 MPa (absolute pressure); preferably, in a range from 1 to 10 MPa.

The propylene oxide production having a good yield is realized preferably by adding either or both of (i) one kind of quinoid compound or a mixture of multiple kinds of quinoid compounds, and/or (ii) one kind of ammonium salt or a mixture of multiple kinds of ammonium salts.

The quinoid compound is grouped into two kinds, i.e. the p-quinoid compound and the o-quinoid compound. The quinoid compound used in the present invention includes both of the above.

Examples of the quinoid compound encompass the p-quinoid compound and a phenantraquinone compound represented by Formula 1 below: 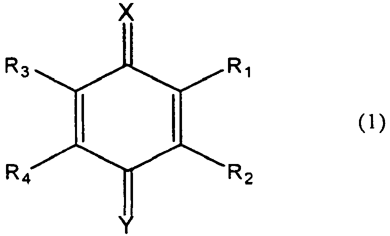 where R₁, R₂, R₃, and R₄ are a hydrogen atom, or either adjacent R₁ and R₂ or adjacent R₃ and R₄ independently bond with each other at their terminal ends, thereby forming, in combination with the carbon atoms of the quinone with which they are bonded, either a benzene ring or a naphthalene ring, which benzene ring or a naphthalene ring being substituted or unsubstituted with an alkyl group or a hydroxyl group, and X and Y are independently or identically either an oxygen atom or an NH group.

Examples of the compound represented by Formula 1 encompass (i) a quinone compound (1A), where R₁, R₂, R₃, and R₄ are a hydrogen atom, and both X and Y are an oxygen atom in Formula 1, (ii) a quinonimine compound (1B), where R₁, R₂, R₃, and R₄ are a hydrogen atom, and X and Y are an oxygen atom and an NH group respectively in Formula 1, and (iii) a quinonediimine compound (1C), where R₁, R₂, R₃, and R₄ are a hydrogen atom, and both X and Y are an NH group in Formula 1. The quinoid compound of Formula 1 encompasses an anthraquinone compound represented by Formula (2) below. 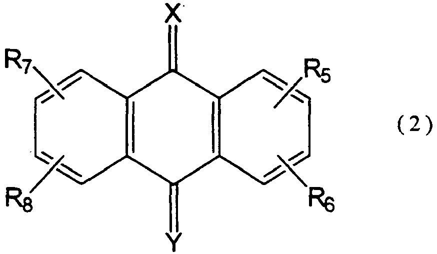 where X and Y are as defined in Formula 1; R₅, R₆, R₇, and R₈ are independently or identically a hydrogen atom, a hydroxyl group, or an alkyl group (e.g. a C₁-C₅ alkyl group such as methyl, ethyl, propyl, butyl, and pentyl).

In Formulae 1 and 2, X and Y are preferably an oxygen atom. The quinoid compound including oxygen atoms at X and Y in Formula 1 is particularly referred to as a quinone compound or a p-quinone compound. The quinoid compound including oxygen atoms at X and Y in Formula 2 is further particularly referred to as an anthraquinone compound.

Examples of a dihydro derivative of the quinoid compound encompass the compounds represented by Formulae 3 and 4 below, i.e. dihydro derivatives of the compounds represented by Formulae 1 and 2. 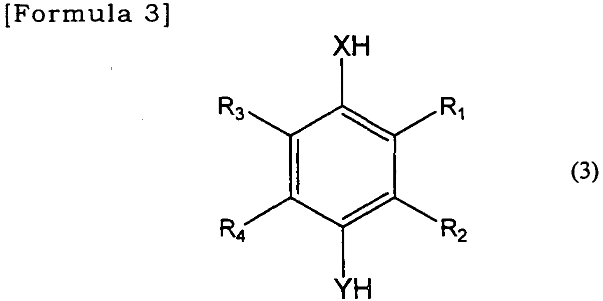 where R₁, R₂, R₃, R₄, X and Y are as defined in Formula 1. 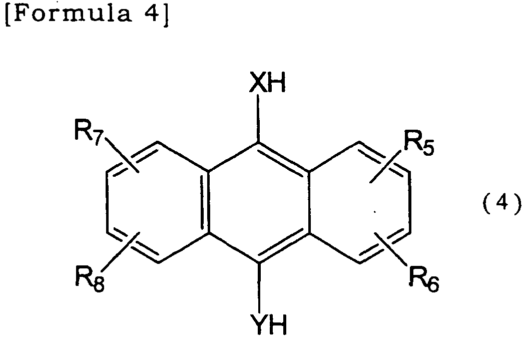 where X, Y, R₅, R₆, R₇, and R₈ are as defined in Formula 2.

In Formulae 3 and 4, X and Y are preferably an oxygen atom. The dihydro derivative of the quinoid compound including oxygen atoms at X and Y in Formula 3 is particularly referred to as a dihydroquinone compound or a dihydro p-quinone compound. The dihydro derivative of the quinoid compound including oxygen atoms at X and Y in Formula 4 is further particularly referred to as a dihydro anthraquinone compound.

Examples of the phenantraquinone compound encompass the p-quinoid compound such as 1,4-phenantraquinone, and the o-quinoid compound such as 1,2-, 3,4-, and 9,10-phenantraquinone.

Specific examples of the quinone compound encompass: benzoquinone; naphthoquinone; anthraquinone; a 2-alkylanthraquinone compound such as 2-ethylanthraquinone, 2-t-butylanthraquinone, 2-amylanthraquinone, 2-methylanthraquinone, 2-butylanthraquinone, 2-t-amylanthraquinone, 2-isopropylanthraquinone, 2-s-butylanthraquinone, and 2-s-amylanthraquinone; 2-hydroxyanthraquinone; a polyalkylanthraquinone compound such as 1,3-diethylanthraquinone, 2,3-dimethylanthraquinone, 1,4-dimethylanthraquinone, and 2,7-dimethylanthraquinone, polyhydroxyanthraquinone such as 2,6-dihydroxyanthraquinone; naphthoquinone; a mixture of the above.

Preferable quinoid compounds include the anthraquinone and the 2-alkylanthraquinone compound (i.e. in Formula 2, X and Y represent oxygen atoms; and R₅ represents a 2-substituted alkyl group, R₆ represents hydrogen, and R₇ and R₈ represent hydrogen atoms). Preferable dihydro derivatives of the quinoid compound include the dihydro derivatives of the above preferable quinoid compounds.

One example of a method for adding the quinoid compound or the dihydro derivative of the quinoid compound (hereinafter referred to as a quinoid compound derivative) into a reaction solvent is carried out by first dissolving the quinoid compound derivative in a liquid phase and then subjecting the resulting solution to the reaction. Alternatively, a compound obtained by hydrogenation of the quinoid compound, e.g. hydroquinone or 9,10-anthracenediol, is first added to the liquid phase. Subsequently, oxidation of the above compound with oxygen in the reactor provides the quinoid compound.

The quinoid compound that may be used for the present invention, including the quinoid compounds shown herein as examples, may become the dihydro forms of partly hydrogenated quinoid compound, depending on reaction conditions; these compounds may also be used.

The quinoid compound is generally dissolved in the acetonitrile solvent before being supplied to the reactor. A lower limit of the quinoid compound to be fed is generally 1×10⁻⁷ mol or more per mol of the propylene to be fed, preferably 1×10⁻⁶ mol or more per mol of the propylene to be fed. An upper limit of the quinoid compound to be fed depends on solubility of the quinoid compound in the solvent; generally, it is 1 mol or more per mol of the propylene to be fed, and preferably 0.1 mol or more per mol of the propylene to be fed.

Examples of the ammonium salt encompass a salt of ammonium, alkylammonium, or alkyl aryl ammonium; specifically, a salt of (i) an anion selected from the group consisting of sulfate ion; hydrogensulfate ion; carbonate ion; hydrogen carbonate ion; phosphate ion; hydrogenphosphate ion; dihydrogenphosphate ion; hydrogenpyrophosphate ion; pyrophosphate ion; halogen ion; nitrate ion; hydroxide ion; and C₁-C₁₀ carboxylate ion; and (ii) a cation selected from the group consisting of ammonium; alkylammonium; and alkyl aryl ammonium.

Examples of the C₁-C₁₀ carboxylate ion encompass: acetate ion; formate ion; acetate ion; propionate ion; butyrate ion; valerate ion; caproate ion; caprylate ion; caprate ion, and benzoate ion.

Examples of the alkylammonium encompass tetramethylammonium, tetraethylammonium, tetra-n-propylammonium, tetra-n-butylammonium, and cetyltrimethylammonium.

Preferable salts of the ammonium, the alkylammonium, or the alkyl aryl ammonium are: ammonium sulfate; ammonium hydrogen sulfate; ammonium carbonate; ammonium hydrogencarbonate; diammonium hydrogen phosphate; ammonium dihydrogenphosphate; ammonium phosphate; ammonium hydrogen pyrophosphate; ammonium pyrophosphate; ammonium chloride; inorganic acid ammonium such as ammonium nitrate; and ammonium (C₁-C₁₀)carboxylate such as ammonium acetate, ammonium benzoate or the like. Among the above, the ammonium dihydrogenphosphate, the diammonium hydrogen phosphate, the ammonium phosphate, and ammonium benzoate are preferable. The ammonium salt in the reaction liquid stabilizes hydrogen peroxide, attains a high concentration of hydrogen peroxide, and decreases production rates of propane and propylene glycol. In particular, control of the solvent pH by adding the ammonium salt significantly decreases the production rates of propane and propylene glycol. In this case, a pH of the solvent is preferably 7.7 or greater. An upper limit of the pH is generally 12.0, and preferably 10; more preferably, 9.0. The pH is determined by measurement of electrode potential, at a temperature of 20°C, across (i) a silver/silver chloride standard electrode that contains an internal solution formed of an aqueous solution having 4 mol/l potassium chloride, and (ii) a silver/silver chloride indicator electrode that contains an internal solution formed of acetate buffer solution, both of the electrodes being immersed in the acetonitrile-water mixture solvent used for the reaction.

The ammonium salt is generally dissolved in a solvent before being fed to the reactor. A lower limit of the ammonium salt to be fed is generally 1×10⁻⁶ mol or more per mol of the propylene to be fed, preferably 1×10⁻⁵ mol or more per mol of the propylene to be fed. An upper limit of the ammonium salt to be fed depends on solubility of the ammonium salt in the solvent; it is generally 2 mol or more per mol of the propylene to be fed, preferably 0.2 mol or more per mol of the propylene to be fed.

The resultant reaction mixture after the reaction is passed through: a gas-liquid separation tower; a solvent recovery tower; a crude propylene oxide separation tower; a propane separation tower; and a solvent refinery tower, so as to be separated into: crude propylene oxide; gas formed mainly of hydrogen, oxygen, and nitrogen; recovered propylene; recovered acetonitrile-water solvent; and a recovered anthraquinone compound. For economical reasons, it is preferable to recycle the recovered propylene, the recovered acetonitrile-water solvent, and the recovered anthraquinone to the reactor. If the recovered propylene includes propane, cyclopropane, methylacetylene, propadiene, butadiene, butanes, butenes, ethylene, ethane, methane or hydrogen, then the recovered propylene may be purified by separation so as to be recycled, where necessary.

If the recovered acetonitrile-water mixture solvent includes compounds generated as a byproduct(s) of the reaction which substance has a boiling point close to an azeotropic temperature of the acetonitrile-water solvent, e.g. acetone, acrylonitrile, oxazole, allyl alcohol, propionitrile propanol, 2,4-dimethyloxazoline, and 2,5-dimethyloxazoline, then the recovered mixture solvent may be purified by separation before reuse, where necessary. If the recovered anthraquinone includes compounds generated as a byproduct(s) of the reaction which has a boiling point higher than the azeotropic temperature of the acetonitrile-water solvent, e.g. water, acetonitrile, an anthracene compound, an anthrahydroquinone compound, a tetrahydroanthraquinone compound, propylene glycol, acetamide, N-(2-hydroxypropane-1-yl)acetamide, and N-(1-hydroxypropane-2-yl)acetamide, then the recovered anthraquinone may be purified by separation before reuse, where necessary.

### Examples

The present invention is described below referring to examples; yet, the present invention is not limited to these examples.

### Referential Example 1: Example of Production of the Ti-MWW Precursor

The Ti-MWW precursor used for the reaction was prepared as follows; first, 112 g of tetra-n-butyl orthotitanate (TBOT), 565 g of boric acid, and 410 g of fumed silica (cab-o-sil M7D) were stirred and dissolved in a mixture of 899 g of piperidine and 2402 g of purified water in an autoclave, at room temperature under air atmosphere, so that a gel was prepared. Then, the gel was stirred for 1.5 hours, and the autoclave was sealed up thereafter. Subsequently, the temperature of the gel was increased over 8 hours while being stirred further and then underwent hydrothermal synthesis by being maintained at 160°C for 120 hours, whereby a suspension was obtained. The suspension thus obtained was filtered, and then a filter cake thereof was washed with water so that the filtrate is adjusted to around pH 10. Next, a filter cake of the suspension was dried at 50°C, whereby water-containing white powder was obtained. Fifteen gram of the powder thus obtained was mixed with 750ml 2N nitric acid and heated for 20 hours under reflux. The resultant mixture was filtered, washed with water to be adjusted to around neutrality, and dried sufficiently at 50°C, whereby 11 g of white powder was obtained. The white powder was analyzed by use of an X-ray diffraction apparatus involving Cu K-alpha radiation thereby determining an X-ray diffraction pattern thereof. The analysis proved that the white powder was a Ti-MWW precursor. According an ICP emission spectrochemical analysis, a titanium content therein was 1.65% by weight.

### Referential Example 2: Example of Production of the Ti-MWW

The Ti-MWW precursor obtained in Referential Example 1 was calcinated at 530°C for 6 hours, whereby Ti-MWW catalyst powder was obtained. As in Referential Example 1, measurement of an X-ray diffraction pattern of the powder thus obtained proved that the powder had MWW structure. According to the ICP emission spectrochemical analysis, a titanium content therein was 1.77% by weight.

### Referential Example 3: Example of Production of the palladium-carrying Ti-MWW Precursor

A 300ml aqueous solution containing 0.0847 mmol of palladium colloid was prepared in a 1L-flask. Then, 9 g of the Ti-MWW precursor obtained in Referential Example 1 was added to the aqueous solution, which was then stirred for 8 hours. After the stirring, water was removed by a rotary evaporator, and a resultant reaction mixture was dried in a vacuum at 80°C for 8 hours. Subsequently, obtained catalyst powder was washed with 1 liter of water, and dried in a vacuum for 8 hours again, whereby a palladium-carrying Ti-MWW precursor was obtained. According to the ICP emission spectrochemical analysis, a palladium content therein was 0.11% by weight.

### Referential Example 4: Example of Production of the palladium-carrying Ti-MWW

A 600ml aqueous solution containing 0.047 mmol of teraammine palladium chloride was prepared in a 1 L-liter recovery flask. Then, 5 g of the Ti-MWW obtained in Referential Example 2 was added to the aqueous solution, which was then stirred for 8 hours. After the stirring, water therein was removed by a rotary evaporator, and a resultant reaction mixture was dried in a vacuum at 80°C for 8 hours. Subsequently, catalyst powder thus obtained was calcinated at 300°C for 6 hours under nitrogen atmosphere, whereby palladium-carrying Ti-MWW was obtained. According to the ICP emission spectrochemical analysis, a palladium content therein was 0.10% by weight.

### Referential Example 5: Silylation of the Ti-MWW Precursor

15g of the Ti-MWW precursor obtained in Referential Example 1 and 175 ml of toluene were mixed, and 11 g of 1,1,1,3,3,3-hexamethyldisilazane was added to a mixture thereof as a silylating agent. Silylation was thereby performed by refluxing a resultant reaction mixture for 4 hours (oil bath temperature: 120°C; internal temperature of the reaction mixture: 110°C). After the refluxing, a resultant reaction mixture was filtered for removal, washed with water, and dried in a vacuum at 150°C, whereby a silylated Ti-MWW precursor was obtained.

### Example 1

In a 300cc autoclave, (i) 131 g of acetonitrile-water solvent (water:acetonitrile = 30:70 by weight ratio), (ii) 2.28 g of a Ti-MWW catalyst, and (iii) 0.74 g of activated carbon carrying 1% of palladium were charged. Then, a pressure in the autoclave was set at 4 MPa (absolute pressure) under nitrogen atmosphere, while a temperature inside the autoclave was kept at 60°C by circulating hot water in a jacket of the autoclave. Subsequently, (i) 195 Nl/h of a mixture of gases containing: 2.6% by volume of hydrogen; 8.6% by volume of oxygen; and 88.7% by volume of nitrogen, (ii) 87.4 g/h of acetonitrile-water solvent (water:acetonitrile = 30:70 by weight ratio) containing 0.7 mmol/kg of anthraquinone and 0.7 mmol/kg of ammonium dihydrogen phosphate, and (iii) 29.2 g/h of liquid propylene containing 0.4% by volume of propane were continuously fed into the autoclave. The temperature and the pressure were maintained at 60°C and 4 MPa respectively during the reaction. The Ti-MWW catalyst and the palladium-carrying activated carbon catalyst, both being in a solid phase, were filtered off with a sintered filter. After the pressure was set back to an atmospheric pressure, the reaction mixture was subjected to gas-liquid separation, whereby liquid and gas thereof were continuously taken out. After 4.5 hours, the reaction liquid and gas were sampled simultaneously, and each of samples was analyzed by gas chromatography; which revealed that the reaction gas at the outlet included 684 mmol/ h of oxygen and 87 mmol/h of hydrogen. Formation rates of propylene oxide and propane are shown in Table 1.

### Example 2

In a 300cc autoclave, (i) 131 g of acetonitrile-water solvent (water:acetonitrile = 40:60 by weight ratio), (ii) 2.28 g of a Ti-MWW catalyst, and (iii) 0.74 g of activated carbon carrying 1% of palladium were charged. Then, a pressure in the autoclave was set at 4 Mpa (absolute pressure) under nitrogen atmosphere, while a temperature inside the autoclave was kept at 60°C by a jacket thereof in which hot water was circulated. Subsequently, (i) 196 N1/h of a mixture of gases containing: 2.6% by volume of hydrogen; 8.5% by volume of oxygen; and 88.9% by volume of nitrogen, (ii) 87.4 g/h of acetonitrile-water solvent (water:acetonitrile = 40:60 by weight ratio) containing 0.7 mmol/kg of anthraquinone and 0.7 mmol/kg of ammonium dihydrogen phosphate, and (iii) 18.0 g/h of liquid propylene containing 0.4% by volume of propane were continuously fed into the autoclave. The temperature and the pressure were maintained at 60°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and gas were sampled simultaneously, and each of the samples was analyzed by gas chromatography; which revealed that the reaction gas at the outlet included 673 mmol/h of oxygen and 91 mmol/h of hydrogen. Formation rates of propylene oxide and propane are shown in Table 1.

### Example 3

In a 300cc autoclave, (i) 131 g of acetonitrile-water solvent (water:acetonitrile = 20:80 by weight ratio), (ii) 2.28 g of a Ti-MWW catalyst, and (iii) 0.74 g of activated carbon carrying 1% of palladium were charged. Then, a pressure in the autoclave was set to 4 MPa (absolute pressure) under nitrogen atmosphere, while a temperature inside the autoclave was kept at 60°C by a jacket thereof in which hot water was circulated. Subsequently, (i) 196.9 N1/h of a mixeture of gases containing: 2.6% by volume of hydrogen; 8.4% by volume of oxygen; and 89.0% by volume of nitrogen, (ii) 86.6 g/h of acetonitrile-water solvent (water:acetonitrile = 20:80 by weight ratio) containing 0.7 mmol/kg of anthraquinone and 0.7 mmol/kg of ammonium dihydrogen phosphate, and (iii) 36.9 g/h of liquid propylene containing 0.4% by volume of propane were continuously fed into the autoclave. The temperature and the pressure were maintained at 60°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas were continuously taken out. After 4.5 hours, the reaction liquid and gas were sampled simultaneously, and each of the samples was analyzed by gas chromatography, which revealed that the reaction gas at the outlet included645 mmol/h of oxygen and 75 mmol /h of hydrogen. Formation rates of propylene oxide and propane are shown in Table 1.

### Example 4

In a 300cc autoclave, (i) 131 g of acetonitrile-water solvent (water:acetonitrile = 0:100 by weight ratio), (ii) 2.28 g of a Ti-MWW catalyst, and (iii) 0.74 g of activated carbon carrying 1% of palladium were charged. Then, a pressure in the autoclave was set at 4 MPa (absolute pressure) under nitrogen atmosphere, while a temperature inside the autoclave was kept at 60°C by a jacket thereof in which hot water was circulated. Subsequently, (i) 188.8 N1/h of a mixture of gases containing: 2.6% by volume of hydrogen; 8.4% by volume of oxygen; and 89.0% by volume of nitrogen, (ii) 86.6 g/h of acetonitrile-water solvent (water:acetonitrile = 0 : 100 by weight ratio) containing 0.7 mmol/kg of anthraquinone and 0.7 mmol/kg of ammonium dihydrogen phosphate, and (iii) 31.8 g/h of liquid propylene containing 0.4% by volume of propane were continuously fed into the autoclave. The temperature and the pressure were maintained at 60°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and gas were sampled simultaneously, and each of the samples was analyzed by gas chromatography, which revealed that the reaction gas at the outlet included 650 mmol/h of oxygen and 62 mmol/h of hydrogen. Formation rates of propylene oxide and propane are shown in Table 1.

### Example 5

In a 300-cc autoclave, (i) 131 g of acetonitrile-water solvent (water:acetonitrile = 40:60 by weight ratio), (ii) 2.28 g of a Ti-MWW catalyst, and (iii) 0.74 g of activated carbon carrying 1% of palladium were charged. Then, a pressure in the autoclave was set at 4 MPa (absolute pressure) under nitrogen atmosphere, while a temperature inside the autoclave was kept at 60°C by a jacket thereof in which hot water was circulated. Subsequently, (i) 203.6 N1/h of a mixture of gases containing: 2.7% by volume of hydrogen; 8.5% by volume of oxygen; and 88.9% by volume of nitrogen, (ii) 81.4 g/h of acetonitrile-water solvent (water:acetonitrile = 40:60 by weight ratio) containing 0.7 mmol/kg of anthraquinone and 0.7 mmol/kg of ammonium dihydrogen phosphate, and (iii) 35.2 g/h of liquid propylene containing 0.4% by volume of propane were continuously fed into the autoclave. The temperature and the pressure were maintained at 60°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and gas were sampled simultaneously, and each of the samples was analyzed by gas chromatography, which revealed that the reaction gas at an outlet included 705 mmol/h of oxygen and 108 mmol/h of hydrogen. Formation rates of propylene oxide and propane are shown in Table 1.

### Example 6

In a 300-cc autoclave, (i) 131 g of acetonitrile-water solvent (water:acetonitrile = 20:80 by weight ratio), (ii) 2.28 g of a Ti-MWW catalyst, and (iii) 1.06 g of activated carbon carrying 1% of palladium were charged. Then, a pressure in the autoclave was set at 4 MPa (absolute pressure) under nitrogen atmosphere, while a temperature inside the autoclave was kept at 50°C by a jacket thereof in which hot water was circulated. Subsequently, (i) 152.8 nl/h of a mixture of gases containing: 3.4% by volume of hydrogen; 3.1% by volume of oxygen; and 93.5% by volume of nitrogen, (ii) 84.0 g/h of acetonitrile-water solvent (water:acetonitrile = 20:80 by weight ratio) containing 0.7 mmol/kg of anthraquinone and 0.7 mmol/kg of ammonium dihydrogen phosphate, and (iii) 36.1 g/h of liquid propylene containing 0.4% by volume of propane were continuously fed into the autoclave. The temperature and the pressure were maintained at 50°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and the reaction gas were sampled simultaneously, and each of the samples was analyzed by gas chromatography, which revealed that the reaction gas at the outlet included 99 mmol/h of oxygen and 30 mmol/h of hydrogen. Formation rates of propylene oxide and propane are shown in Table 1.

### Example 7

In a 300cc autoclave, (i) 131 g of acetonitrile-water solvent (water:acetonitrile = 20:80 by weight ratio), (ii) 2.28 g of a Ti-MWW catalyst, and (iii) 1.06 g of activated carbon carrying 1% of palladium were charged. Then, a pressure in the autoclave was set at 4 MPa (absolute pressure) under nitrogen atmosphere, while a temperature inside the autoclave was kept at 50°C by a jacket thereof in which hot water was circulated. Subsequently, (i) 153.2 N1/h of a mixture of gases containing: 3.4% by volume of hydrogen; 2.3% by volume of oxygen; and 93.5% by volume of nitrogen, (ii) 86.6 g/h of acetonitrile-water solvent (water:acetonitrile = 20:80 by weight ratio) containing 0.7 mmol/kg of anthraquinone and 0.7 mmol/kg of ammonium dihydrogen phosphate, and (iii) 35.2 g/h of liquid propylene containing 0.4% by volume of propane were continuously fed into the autoclave. The temperature and the pressure were maintained at 50°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the Reaction mixture was subjected to gas-liquid separation, whereby liquid and gas thereof were continuously taken out. After 4.5 hours, the reaction liquid and gas were sampled simultaneously, and each of the samples was analyzed by gas chromatography, which revealed that the reaction gas at an outlet included 34 mmol/h of oxygen and 24 mmol/h of hydrogen. Formation rates of propylene oxide and propane are shown in Table 1.

### Example 8

In a 300cc autoclave, (i) 131 g of acetonitrile-water solvent (water:acetonitrile = 20:80 by weight ratio), (ii) 2.28 g of a Ti-MWW catalyst, and (iii) 1.06 g of activated carbon carrying 1% of palladium were charged. Then, a pressure in the autoclave was set at 4 MPa (absolute pressure) under nitrogen atmosphere, while a temperature inside the autoclave was kept at 50°C by a jacket thereof in which hot water was circulated. Subsequently, (i) 153.9 N1/h of a mixture of gases containing: 3.9% by volume of hydrogen; 1.9% by volume of oxygen; and 94.1% by volume of nitrogen, (ii) 86.6 g/h of acetonitrile-water solvent (water:acetonitrile = 20:80 by weight ratio) containing 0.7 mmol/kg of anthraquinone and 0.7 mmol/kg of ammonium dihydrogen phosphate, and (iii) 30.9 g/h of liquid propylene containing 0.4% by volume of propane were continuously fed into the autoclave. The temperature and the pressure were maintained at 50°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the Reaction mixture was subjected to gas-liquid separation, whereby liquid and gas thereof were continuously taken out. After 4.5 hours, the reaction liquid and gas were sampled simultaneously, and each of the samples was analyzed by gas chromatography, which revealed that the reaction gas at the outlet included 23 mmol/h of oxygen and 52 mmol/h of hydrogen. Formation rates of propylene oxide and propane are shown in Table 1.

### Example 9

In a 300cc autoclave, (i) 131 g of acetonitrile-water solvent (water:acetonitrile = 30:70 by weight ratio), (ii) 2.28 g of a Ti-MWW catalyst, and (iii) 1.06 g of activated carbon carrying 1% of palladium were charged. Then, a pressure in the autoclave was set at 4 MPa (absolute pressure) under nitrogen atmosphere, while a temperature inside the autoclave was kept at 50°C by a jacket thereof in which hot water was circulated. Subsequently, (i) 144.1 nl/h of a mixture of gases containing: 3.6% by volume of hydrogen; 2.4% by volume of oxygen; and 94.0% by volume of nitrogen, (ii) 84.0 g/h of acetonitrile-water solvent (water:acetonitrile = 30:70 by weight ratio) containing 0.7 mmol/kg of anthraquinone and 0.7 mmol/kg of ammonium dihydrogen phosphate, and (iii) 33.5 g/h of liquid propylene containing 0.4% by volume of propane were continuously fed into the autoclave. The temperature and the pressure were maintained at 50°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and the reaction gas were sampled simultaneously, and each of the samples was analyzed by gas chromatography, which revealed that the reaction gas at the outlet included 59 mmol/h of oxygen and 55 mmol/h of hydrogen. Formation rates of propylene oxide and propane are shown in Table 1.

### Example 10

In a 300cc autoclave, (i) 131 g of acetonitrile-water solvent (water:acetonitrile = 30:70 by weight ratio), (ii) 2.28 g of a Ti-MWW catalyst, and (iii) 1.06 g of activated carbon carrying 1% of palladium were charged. Then, a pressure in the autoclave was set at 4 MPa (absolute pressure) under nitrogen atmosphere, while a temperature inside the autoclave was kept at 60°C by a jacket thereof in which hot water was circulated. Subsequently, (i) 138.7 N1/h of a mixture of gases containing: 3.9% by volume of hydrogen; 2.5% by volume of oxygen; and 93.6% by volume of nitrogen, (ii) 84.0 g/h of acetonitrile-water solvent (water:acetonitrile = 30:70 by weight ratio) containing 0.7 mmol/kg of anthraquinone and 0.7 mmol/kg of ammonium dihydrogen phosphate, and (iii) 28.4 g/h of liquid propylene containing 0.4% by volume of propane were continuously fed into the autoclave. The temperature and the pressure were maintained at 60°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and gas were sampled simultaneously, and each of the samples was analyzed by gas chromatography, which revealed that the reaction gas at the outlet included 53 mmol/h of oxygen and 34 mmol/h of hydrogen. Formation rates of propylene oxide and propane are shown in Table 1.

### Example 11

In a 300cc autoclave, (i) 131 g of acetonitrile-water solvent (water:acetonitrile = 20:80 by weight ratio), (ii) 2.28 g of a Ti-MWW catalyst, and (iii) 1.06 g of activated carbon carrying 1% of palladium were charged. Then, a pressure in the autoclave was set at 4 MPa (absolute pressure) under nitrogen atmosphere, while a temperature inside the autoclave was kept at 50°C by a jacket thereof in which hot water was circulated. Subsequently, (i) 154.8 N1/h of a mixture of gases containing: 3.1% by volume of hydrogen; 8.3% by volume of oxygen; and 88.6% by volume of nitrogen, (ii) 87.4 g/h of acetonitrile-water solvent (water:acetonitrile = 20:80 by weight ratio) containing 0.7 mmol/kg of anthraquinone and 0.7 mmol/kg of ammonium dihydrogen phosphate, and (iii) 32.6 g/h of liquid propylene containing 0.4% by volume of propane were continuously fed into the autoclave. The temperature and the pressure were maintained at 50°C and 4 MPa respectively during the reaction. The Ti-MWW catalyst and the palladium-carrying activated carbon catalyst, both being in a solid phase, were filtered by a sintered filter. After the pressure was set back to an atmospheric pressure, the reaction mixture was subjected to gas-liquid separation, whereby liquid and gas thereof were continuously taken out. After 4.5 hours, the reaction liquid and gas were sampled simultaneously, and each of the samples was analyzed by gas chromatography, which revealed that the reaction gas at the outlet included 484 mmol/h of oxygen and 78 mmol/h of hydrogen. Formation rates of propylene oxide and propane are shown in Table 1.

**Table 1**

| No. | Ex. 1 | Ex. 2 | Ex. 3 | Ex. 4 | Ex. 5 | Ex. 6 | Ex. 7 | Ex. 8 | Ex. 9 | Ex. 10 | Ex. 11 |
|---|---|---|---|---|---|---|---|---|---|---|---|
| Water/ Acetonitrile (weight ratio) | 30/70 | 40/60 | 20/80 | 0/100 | 40/60 | 20/80 | 20/80 | 20/80 | 30/70 | 30/70 | 20/80 |
| Propylene / Acetonitrile -water solvent (weight ratio) | 0.25 | 0.17 | 0.30 | 0.27 | 0.30 | 0.30 | 0.29 | 0.26 | 0.29 | 0.25 | 0.27 |
| Oxygen / Hydrogen at outlet of reactor (volume ratio) | 7.9 | 7.4 | 8.6 | 10.5 | 6.5 | 3.3 | 1.4 | 0.4 | 1.1 | 1.6 | 6.2 |
| Propylene oxide formation rate (mmol/h) | 53.2 | 39.5 | 50.4 | 28.8 | 40.3 | 60.0 | 63.3 | 59.1 | 53.0 | 49.0 | 65.0 |
| Propane formation rate (mmol/h) | 6.0 | 1.9 | 11.3 | 32.5 | 3.3 | 7.0 | 7.4 | 8.2 | 0.55 | 0.77 | 17.2 |

| | | | | | | | | | | | |
|---|---|---|---|---|---|---|---|---|---|---|---|
| (In Table 1, Ex. refers to Example | | | | | | | | | | | |

### Example 12

In a 300cc autoclave, (i) 131 g of acetonitrile-water solvent (water:acetonitrile = 30:70 by weight ratio), (ii) 2.28 g of Ti-MWW, and (iii) 0.198 g of activated carbon carrying 1.29% of palladium were charged. Then, a pressure in the autoclave was set at 4 MPa in [absolute pressure] under nitrogen atmosphere, while a temperature inside the autoclave was kept at 50°C by a jacket thereof in which hot water was circulated. Subsequently, (i) 146 N1/h of a mixture of gases containing: 3.6% by volume of hydrogen; 2.1% by volume of oxygen; and 94.3% by volume of nitrogen, (ii) 90 g/h of acetonitrile-water solvent (water:acetonitrile = 30:70 by weight ratio) containing 0.7 mmol/kg of anthraquinone and 0.7 mmol/kg of ammonium dihydrogen phosphate, and (iii) 36 g/h of liquid propylene containing 0.4% by volume of propane were continuously fed to the autoclave. The temperature and the pressure were maintained at 50°C and 4 MPa respectively during the reaction. The Ti-MWW and the palladium-carrying activated carbon catalyst, both being in a solid phase, were filtered by a sintered filter. After the reaction mixture was subjected to gas-liquid separation, the pressure was set back to an atmospheric pressure, whereby liquid and gas thereof were continuously taken out. After 6 hours, the reaction liquid and the reaction gas were sampled simultaneously, and each of the samples was analyzed by gas chromatography. Formation rates of propylene oxide and propylene glycol were 44 mmol/h and 3.1 mmol/h respectively.

### Example 13

An experiment was carried out in the same manner as in Example 12, except that 2.28 g of a Ti-MWW precursor was used in place of the Ti-MWW. Formation rates of propylene oxide and propylene glycol were 61 mmol/h and 11 mmol/h respectively.

### Example 14

An experiment was carried out in the same manner as in Example 12, except that 2.28 g of a Ti-MWW precursor having a silylated surface was used in place of the Ti-MWW. Formation rates of propylene oxide and propylene glycol were 56 mmol/h and 4.3 mmol/h respectively.

### Example 15

An experiment was carried out in the same manner as in Example 12, except that 1.98 g of the Ti-MWW precursor carrying 0.1% by weight of palladium was used in place of the Ti-MWW and the activated carbon carrying 1.29% of palladium. Formation rates of propylene oxide and propylene glycol were 66 mmol/h and 4.2 mmol/h respectively.

### Example 16

An experiment was carried out in the same manner as in Example 12, except that 1.98 g of Ti-MWW carrying 0.1% by weight of palladium was used in place of the Ti-MWW and the activated carbon carrying 1.29% of palladium. Formation rates of propylene oxide and propylene glycol were 52 mmol/h and 3.6 mmol/h respectively.

### Example 17

An experiment was carried out in the same manner as in Example 12, except that an amount of the activated carbon carrying 1.29% of palladium was 1.056 g. Formation rates of propylene oxide and propylene glycol were 44 mmol/h and 2.4 mmol/h respectively.

### Example 18

An experiment was carried out in the same manner as in Example 12, except that an amount of the activated carbon carrying 1.29% of palladium was 0.528 g. Formation rates of propylene oxide and propylene glycol were 42 mmol/h and 2.5 mmol/h respectively.

### Example 19

An experiment was carried out in the same manner as in Example 12, except that an amount of the activated carbon carrying 1.29% of palladium was 0.264 g. Formation rates of propylene oxide and propylene glycol were 44 mmol/h and 2.4 mmol/h respectively.

### Example 20

An experiment was carried out in the same manner as in Example 12, except that an amount of the activated carbon carrying 1.29% of palladium was 0.132 g. Formation rates of propylene oxide and propylene glycol were 8.6 mmol/h and 1.4 mmol/h respectively.

### Example 22

An experiment was carried out in the same manner as in Example 12, except that 1.98 g of a Ti-MWW precursor carrying 0.12% by weight of palladium was used in place of the Ti-MWW and the activated carbon carrying 1.29% of palladium. Formation rates of propylene oxide and propylene glycol were 67 mmol/h and 4.8 mmol/h respectively.

### Example 23

An experiment was carried out in the same manner as in Example 12, except that 1.98 g of a Ti-MWW precursor carrying 0.05% by weight of palladium was used in place of the Ti-MWW and the activated carbon carrying 1.29% of palladium. Formation rates of propylene oxide and propylene glycol were 55 mmol/h and 4.7 mmol/h respectively.

### Example 24

An experiment was carried out in the same manner as in Example 12, except that 1.98 g of a Ti-MWW precursor carrying 0.025% by weight of palladium was used in place of the Ti-MWW and the activated carbon carrying 1.29% of palladium. Formation rates of propylene oxide and propylene glycol were 6.6 mmol/h and 1.3 mmol/h respectively.

### Example 25

In a 300cc autoclave, (i) 131 g of acetonitrile-water solvent (water:acetonitrile = 30:70 by weight ratio), (ii) 2.28 g of a Ti-MWW precursor, and (iii) 0.198 g of activated carbon carrying 1.29% of palladium were charged. Then, a pressure in the autoclave was set at 4 MPa in absolute pressure under nitrogen atmosphere, while a temperature inside the autoclave was kept at 50°C by a jacket thereof in which hot water was circulated. Subsequently, (i) 146 N1/h of a mixture of gases containing: 3.6% by volume of hydrogen; 2.1% by volume of oxygen; and 94.3% by volume of nitrogen, (ii) 90 g/h of acetonitrile-water mixture solvent (water:acetonitrile = 30:70 by weight ratio) containing 0.7 mmol/kg of anthraquinone, and (iii) 36 g/h of liquid propylene containing 0.4 volume percent of propane according to gas chromatography analysis, were continuously fed into the autoclave. A pH of the acetonitrile-water mixture solvent supplied to a reactor was 7.1. The temperature and the pressure were maintained at 50°C and 4 MPa respectively during the reaction. The Ti-MWW precursor and the palladium-carrying activated carbon catalyst, both being in a solid phase, were filtered by a sintered filter. After the reaction mixture was subjected to gas-liquid separation, the pressure was set back to an atmospheric pressure, whereby liquid and gas thereof were continuously taken out. After 6 hours, the reaction liquid and the reaction gas were sampled simultaneously, and each of the samples was analyzed by gas chromatography. Further, a concentration of hydrogen peroxide contained in the reaction liquid was determined by titration with potassium permanganate. Formation rates of propylene oxide, propylene glycol, and propane were 53 mmol/h, 5.9 mmol/h, and 6.3 mmol/h respectively. The concentration of the hydrogen peroxide contained in the reaction liquid was 0.03%.

### Example 26

An experiment was carried out in the same manner as in Example 25, except that water-acetonitrile mixture solvent (water:acetonitrile = 30:70 by weight ratio) containing 0.7 mmol/kg of anthraquinone and 3.0 mmol/kg of ammonium dihydrogen phosphate was used, in place of the acetonitrile-water mixture solvent containing 0.7 mmol/kg of anthraquinone. A pH of the solvent fed into a reactor was 6.0. Formation rates of propylene oxide, propylene glycol, and propane were 55 mmol/h, 5.3 mmol/h, and 5.5 mmol/h respectively. A concentration of hydrogen peroxide contained in a reaction liquid was 0.07%.

### Example 27

An experiment was carried out in the same manner as in Example 26, except that 3.0 mmol/kg of ammonium benzoate was used, in place of 3.0 mmol/kg of the ammonium dihydrogen phosphate. A pH of the solvent fed into a reactor was 7.7. Formation rates of propylene oxide, propylene glycol, and propane were 50 mmol/h, 3.4 mmol/h, and 3.8 mmol/h respectively. A concentration of hydrogen peroxide contained in a reaction liquid was 0.05%.

### Example 28

An experiment was carried out in the same manner as in Example 26, except that 3.0 mmol/kg of diammonium hydrogen phosphate was used, in place of 3.0 mmol/kg of the ammonium dihydrogen phosphate. A pH of the solvent fed into a reactor was 8.2. Formation rates of propylene oxide, propylene glycol, and propane were 52 mmol/h, 2.4 mmol/h, and 4.5 mmol/h respectively. A concentration of hydrogen peroxide contained in a reaction liquid was 0.07%.

### Example 29

An experiment was carried out in the same manner as in Example 26, except that 3.0 mmol/kg of ammonium phosphate was used, in place of 3.0 mmol/kg of the ammonium dihydrogen phosphate. A pH of the solvent fed into a reactor was 8.6. Formation rates of propylene oxide, propylene glycol, and propane were 44 mmol/h, 2.8 mmol/h, and 4.4 mmol/h respectively. A concentration of hydrogen peroxide contained in a reaction liquid was 0.10%.

The method of the present invention realizes efficient production of propylene oxide.

The specific embodiments and examples provided herein should be considered in all aspect as illustrative of the technical concept of the present invention and the present invention should not be construed as limited thereto.

## Claims

1. A method for producing propylene oxide, comprising:
reacting hydrogen, oxygen, and propylene, in an acetonitrile solvent or in a mixture of solvents which include acetonitrile and water, in the presence of a titanosilicate catalyst having a 12 or more-membered oxygen ring pore and a palladium catalyst supported on a carrier, wherein the propylene is supplied to the reaction in the form of liquefied propylene.

2. A method according to claim 1, wherein a weight ratio of water to acetonitrile in the mixture of solvents which include acetonitrile and water is in a range from (i) 50 : 50 to (ii) 0 : 100.

3. A method according to claim 1, wherein a weight ratio of water to acetonitrile in the mixture of solvents which include acetonitrile and water is in a range from (i) 21 : 79 to (ii) 40 : 60.

4. A method according to claim 2 or 3, wherein a feed amount of the mixture of solvents which include acetonitrile and water is in a range from 0.02 to 70 parts per part by weight of a feed amount of the propylene.

5. A method according to claim 2 or 3, wherein a feed amount of the mixture of solvents which include acetonitrile and water is in a range from 0.2 to 20 parts by weight per part by weight of a feed amount of the propylene.

6. A method according to claim 2 or 3, wherein a feed amount of the mixture of solvents which include acetonitrile and water is in a range from 1 to 10 parts by weight per part by weight of a feed amount of the propylene.

7. A method according to claim 1 to 6, wherein a volume ratio of the oxygen to the hydrogen at an outlet of a reactor is not greater than 3.5.

8. A method according to claim 1 to 7, wherein the palladium catalyst supported on the carrier is palladium catalyst supported on activated carbon.

9. A method according to claim 1 to 7, wherein the carrier is titanosilicate.

10. A method according to claim 1 to 9, wherein the titanosilicate catalyst having a 12 or more-membered oxygen ring pore is Ti-MWW .

11. A method according to claim 8, wherein the weight ratio of the palladium to the reaction solvent fed into a reactor is greater than 13 ppm by weight.

12. A method according to claim 9, wherein the weight ratio of the palladium to the reaction solvent fed into a reactor is greater than 4 ppm by weight.

13. A method according to claim 1 to 12, wherein the reaction solvent fed into a reactor is a reaction solvent that contains an ammonium salt.

14. A method according to claim 13, wherein the reaction solvent shows weak basicity.

15. A method according to claim 13 or 14, wherein pH of the reaction solvent is 7.7 or greater.

## Patentansprüche

1. Ein Verfahren zur Herstellung von Propylenoxid, umfassend:
Umsetzen von Wasserstoff, Sauerstoff und Propylen in einem Acetonitril-Lösungsmittel oder in einem Gemisch von Lösungsmitteln, welches Acetonitril und Wasser enthält, in der Gegenwart eines Titansilicatkatalysators mit einer 12- oder mehrgliedrigen Sauerstoffringpore und eines auf einen Träger aufgebrachten Palladiumkatalysators, wobei das Propylen der Reaktion in Form von verflüssigtem Propylen zugeführt wird.

2. Ein Verfahren nach Anspruch 1, wobei ein Gewichtsverhältnis von Wasser zu Acetonitril in dem Gemisch von Lösungsmitteln, welches Acetonitril und Wasser enthält, in einem Bereich von (i) 50 : 50 bis (ii) 0 : 100 liegt.

3. Ein Verfahren nach Anspruch 1, wobei ein Gewichtsverhältnis von Wasser zu Acetonitril in dem Gemisch von Lösungsmitteln, welches Acetonitril und Wasser enthält, in einem Bereich von (i) 21 : 79 bis (ii) 40 : 60 liegt.

4. Ein Verfahren nach Anspruch 2 oder 3, wobei eine Zufuhrmenge des Gemischs von Lösungsmitteln, welches Acetonitril und Wasser enthält, in einem Bereich von 0,02 bis 70 Gewichtsteilen pro Gewichtsteil einer Zufuhrmenge des Propylens liegt.

5. Ein Verfahren nach Anspruch 2 oder 3, wobei eine Zufuhrmenge des Gemischs von Lösungsmitteln, welches Acetonitril und Wasser enthält, in einem Bereich von 0,2 bis 20 Gewichtsteilen pro Gewichtsteil einer Zufuhrmenge des Propylens liegt.

6. Ein Verfahren nach Anspruch 2 oder 3, wobei eine Zufuhrmenge des Gemischs von Lösungsmitteln, welches Acetonitril und Wasser enthält, in einem Bereich von 1 bis 10 Gewichtsteilen pro Gewichtsteil einer Zufuhrmenge des Propylens liegt.

7. Ein Verfahren nach Anspruch 1 bis 6, wobei ein Volumenverhältnis des Sauerstoffs zu dem Wasserstoff an einer Auslassöffnung eines Reaktors nicht größer als 3,5 ist.

8. Ein Verfahren nach Anspruch 1 bis 7, wobei der auf dem Träger aufgebrachte Palladiumkatalysator auf Aktivkohle aufgebrachter Palladiumkatalysator ist.

9. Ein Verfahren nach Anspruch 1 bis 7, wobei der Träger Titansilicat ist.

10. Ein Verfahren nach Anspruch 1 bis 9, wobei der Titansilicatkatalysator mit einer 12-oder mehrgliedrigen Sauerstoffringpore Ti-MWW ist.

11. Ein Verfahren nach Anspruch 8, wobei das Gewichtsverhältnis des Palladiums zu dem Reaktionslösungsmittel, welches in einen Reaktor eingespeist wird, größer als 13 Gewichts-ppm ist.

12. Ein Verfahren nach Anspruch 9, wobei das Gewichtsverhältnis des Palladiums zu dem Reaktionslösungsmittel, welches in einen Reaktor eingespeist wird, größer als 4 Gewichts-ppm ist.

13. Ein Verfahren nach Anspruch 1 bis 12, wobei das Reaktionslösungsmittel, welches in einen Reaktor eingespeist wird, ein Reaktionslösungsmittel ist, das ein Ammoniumsalz enthält.

14. Ein Verfahren nach Anspruch 13, wobei das Reaktionslösungsmittel schwache Basizität aufweist.

15. Ein Verfahren nach Anspruch 13 oder 14, wobei der pH des Reaktionslösungsmittels 7,7 oder höher ist.

## Revendications

1. Procédé de production d'oxyde de propylène, comprenant :
la réaction d'hydrogène, d'oxygène, et de propylène, dans un solvant d'acétonitrile ou dans un mélange de solvants qui comprend de l'acétonitrile et de l'eau, en présence d'un catalyseur de silicate de titane ayant des pores de la taille d'un cycle oxygène à 12 chaînons ou plus et d'un catalyseur de palladium supporté sur un support, dans lequel le propylène est introduit dans la réaction sous la forme de propylène liquéfié.

2. Procédé selon la revendication 1, dans lequel un rapport en poids de l'eau à l'acétonitrile dans le mélange de solvants qui comprend de l'acétonitrile et de l'eau est dans une plage de (i) 50:50 à (ii) 0:100.

3. Procédé selon la revendication 1, dans lequel un rapport en poids de l'eau à l'acétonitrile dans le mélange de solvants qui comprend de l'acétonitrile et de l'eau est dans une plage de (i) 21:79 à (ii) 40:60.

4. Procédé selon la revendication 2 ou 3, dans lequel une quantité introduite du mélange de solvants qui comprend de l'acétonitrile et de l'eau est dans une plage de 0,02 à 70 parties en poids par partie en poids d'une quantité introduite de propylène.

5. Procédé selon la revendication 2 ou 3, dans lequel une quantité introduite du mélange de solvants qui comprend de l'acétonitrile et de l'eau est dans une plage de 0,2 à 20 parties en poids par partie en poids d'une quantité introduite de propylène.

6. Procédé selon la revendication 2 ou 3, dans lequel une quantité introduite du mélange de solvants qui comprend de l'acétonitrile et de l'eau est dans une plage de 1 à 10 parties en poids par partie en poids d'une quantité introduite de propylène.

7. Procédé selon les revendications 1 à 6, dans lequel un rapport en volume de l'oxygène à l'hydrogène à une sortie d'un réacteur n'est pas supérieur à 3,5.

8. Procédé selon les revendications 1 à 7, dans lequel le catalyseur de palladium supporté sur le support est un catalyseur de palladium supporté sur du charbon actif.

9. Procédé selon les revendications 1 à 7, dans lequel le support est le silicate de titane.

10. Procédé selon les revendications 1 à 9, dans lequel le catalyseur de silicate de titane ayant des pores de la taille d'un cycle oxygène à 12 chaînons ou plus est Ti-MWW.

11. Procédé selon la revendication 8, dans lequel le rapport en poids du palladium au solvant de réaction introduit dans un réacteur est supérieur à 13 ppm en poids.

12. Procédé selon la revendication 9, dans lequel le rapport en poids du palladium au solvant de réaction introduit dans un réacteur est supérieur à 4 ppm en poids.

13. Procédé selon les revendications 1 à 12, dans lequel le solvant de réaction introduit dans un réacteur est un solvant de réaction qui contient un sel d'ammonium.

14. Procédé selon la revendication 13, dans lequel le solvant de réaction présente une faible basicité.

15. Procédé selon la revendication 13 ou 14, dans lequel le pH du solvant de réaction est de 7,7 ou plus.
